(19) Europäisches Patentamt
European Patent Office
Office européen des brevets

(11) Publication number: **0 277 596 A2**

(12) **EUROPEAN PATENT APPLICATION**

(21) Application number: **88101266.0**

(51) Int. Cl.4: **C07C 87/52** , C07C 85/24

(22) Date of filing: **28.01.88**

(30) Priority: **03.02.87 US 10354**

(43) Date of publication of application: **10.08.88 Bulletin 88/32**

(84) Designated Contracting States: **BE CH DE FR GB IT LI NL**

(71) Applicant: **AIR PRODUCTS AND CHEMICALS, INC.**
**Route no. 222**
**Trexlertown Pennsylvania 18087(US)**

(72) Inventor: **Burgoyne, William Franklin, Jr.**
**1090C Cold Stream Circle**
**Emmaus, PA 18049(US)**
Inventor: **Dixon, Dale David**
**2216 Parklane Pointe**
**Venice, Fl 34293(US)**

(74) Representative: **Dipl.-Ing. Schwabe, Dr. Dr. Sandmair, Dr. Marx**
**Stuntzstrasse 16**
**D-8000 München 80(DE)**

(54) **Amino-beta-Alkyl styrenes for use in preparing polyurethane/urea systems and polymers.**

(57) The invention relates to a class of amino-β-alkyl styrenes and process for producing such styrenes. More particularly, the aminoalkyl styrenes are represented by the formula:

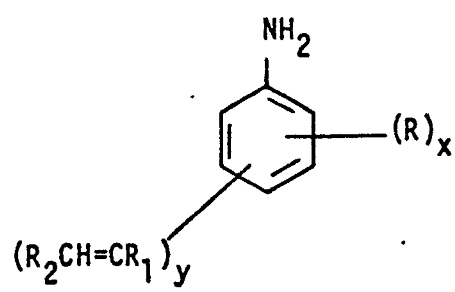

wherein R is H or $C_{1-5}$ alkyl; $R_1$ is H or $C_1$, methyl; $R_2$ is $C_{1-4}$alkyl; or $R_1$ and $R_2$ are combined as a 5 carbon member ring, x is 1 or 2, and y is 1 or 2 when R is H.

The above described amino styrenes have been found to be well suited in forming polyurethane/urea elastomer systems and for providing pendant unsaturation for post curing and polymerization with other monomers.

EP 0 277 596 A2

# AMINO-$\beta$-ALKYL STYRENES FOR USE IN PREPARING POLYURETHANE/UREA SYSTEMS AND POLYMERS

## TECHNICAL FIELD

This invention pertains to alkenylated aromatic amines having particular suitability for post curable, cross-linkable polyurethane/urea elastomer systems and a process for making them.

## BACKGROUND OF THE INVENTION

Aromatic compositions having unsaturated organo substituents are known and have been prepared by alkylation of aromatic compositions with diloefins. Some patents which show the manufacture of aromatic compositions having unsaturated organo substituents include:
U.S. 2,403,963 which discloses the reaction of benzene and butadiene in the presence of a boron trifluoride catalyst. Phenylbutenes are produced.

U.S. 2,471,922 discloses that aromatic hydrocarbons, which include phenols and aromatic halides, can be reacted with 1,3-diolefins to produce alkenyl derivatives. Examples of low-boiling 1,3-diolefins which can be used in the alkenylation include 1,3-butadiene, 1,3-pentadiene and so forth. Catalyst systems included boron trifluoride and boron trifluoride-phosphoric acid systems.

U.S. 3,865,889 discloses the preparation of an alkenylated aromatic hydrocarbon such as those obtained by reacting butadiene with an alkylbenzene, e.g., toluene or xylene. An alkali metal promoter is used to catalyze the reaction.

U.S. 2,843,565 discloses the production of butenyl-phenol-aldehyde resins which involves the reaction of an alkenylphenol, with formaldehyde. Conjugated dienes such as piperylene, cyclopentadiene, 1-chloro-2-methyl-butadiene are reacted with phenols in the presence of phosphoric acid catalyst and the reaction product then reacted with formaldehyde.

West German 1,079,628 discloses the preparation of cyclopentenyl-substituted aromatic amines by reacting cyclopentadiene with primary, secondary or tertiary aromatic amine substrates at elevated temperature in the presence of bleaching earths. Aniline, N-methylaniline, chloroaniline, and phenylenediamine are suggested as candidates.

European Patent 0082258 discloses various meta-phenylenediamines having one or two benzyl rings having lower reactivity due to steric hindrance. Steric hindrance is effected through the use of an aromatic substituent which also may be substituted with various groupings. An example of an amine is 4,6-bis-($\alpha$,$\alpha$-dimethylbenzyl)-1,3-phenylenediamine prepared by reacting $\alpha$-methylstyrene with meta-phenylenediamine.

## SUMMARY OF THE INVENTION

This invention pertains to an alkenylated aromatic amine and particularly to mono-alkenylated aniline. In contrast to prior art aromatic amine compositions, the organo group contains carbon-carbon unsaturation and is conjugated with the aromatic ring. These aromatic amine compositions are represented by the formula:

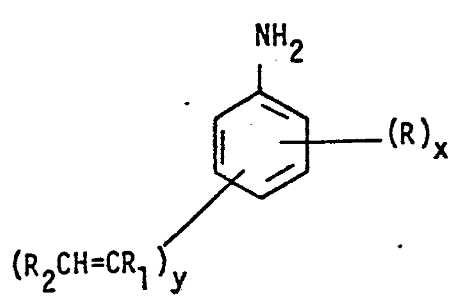

wherein R is H or $C_{1-5}$ alkyl; $R_1$ is H or $C_1$ methyl; $R_2$ is $C_{1-4}$alkyl; or $R_1$ and $R_2$ are combined as a 5 carbon member ring, x is 1 or 2, and y is 1 or 2 when R is H.

There are several advantages associated with the specific compositions of this invention and these advantages include:

- a composition which, having both an amino functionality and styrenic functionality, may be used for preparing prepolymers end-capped with unsaturation suitable for use in post curing procedures;
- an ability, in the form an anilium salt, to reactively add to other unsaturated monomers;

DETAILED DESCRIPTION OF THE INVENTION

The compounds of this invention, as indicated above, are represented by the formula:

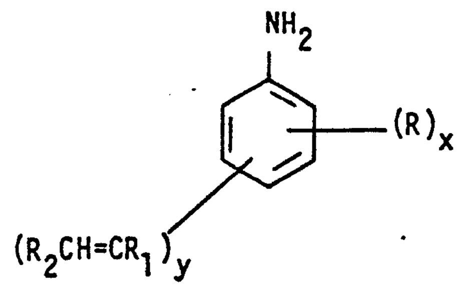

wherein R is H or $C_{1\text{-}5}$ alkyl, $R_1$ is H or $C_1$ methyl; $R_2$ is $C_{1\text{-}4}$alkyl; or $R_1$ and $R_2$ are combined as 5 carbon member ring, x is 1 or 2, and y is 1 or 2 when R is H.

The structural formula has been written to reflect that stereo chemistry exists in the reaction product and $R_1$ may be cis or trans to $R_2$.

The compounds of this invention are synthesized by a two-step process consisting of alkenylation of an arylamine with a diene followed by isomerization of the double bond in the alkenylation product. The overall process is generalized in scheme 1.

Scheme 1

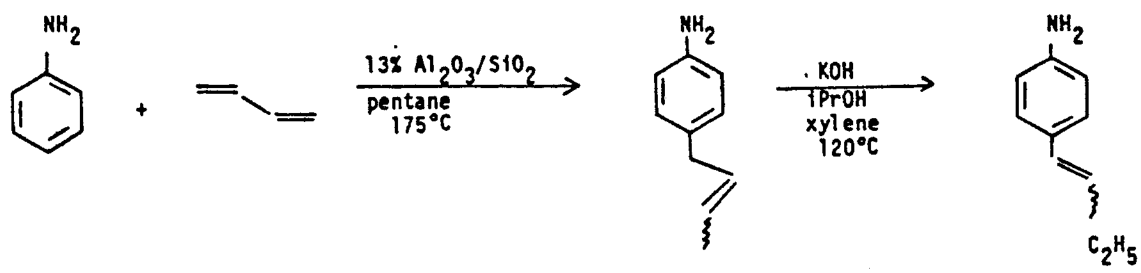


The first step (i.e., reaction of an arylamine with a conjugated diene) allows introduction of at least one alkenyl group onto the aromatic ring. The alkenylation is effected best by use of a solid acid catalyst.

Much of the art describing the manufacture of alkenylated aromatic hydrocarbons uses a homogenous catalyst system, e.g., boron trifluoride or boron trifluoride-phosphoric acid mixture or weakly acidic heterogeneous catalyst systems. Other art in the manufacture of alkylated aromatic amines use bleaching earths, clays, montmorillonite and alkali metal ion exchanged phenols and alkylated aromatic amines, it is our belief alkenylated aromatic amines are best prepared using silica-alumina (silica content of at least 70% by weight) or crystalline molecular sieves which are solid phase and have an acidity factor of at least 0.3 and preferably in excess of 0.8 as the catalyst. The acidity factor is a measurement of acidity of the zeolite catalyst and involves contact of the catalyst with ammonia under adsorption conditions followed by desorption. More particularly, one gram of catalyst is contacted with ammonia at room temperature and then desorbed by heating to a temperature from ambient to 200°C at a rate of 10° per minute, then holding at 200°C for two hours. The amount of ammonia irreversibly adsorbed by one gram at 200°C is indicative of acidity and indicative of the strength of the amine/acid bond. The acidity factor then is the amount of ammonia irreversibly adsorbed expressed in millimoles per gram of catalyst at 200°C and as stated this

level should be at least 0.3 and preferably 0.8 millimoles ammonia per gram of catalyst.

Zeolites which can be utilized for alkenylation of toluenediamines include X, Y, faujasite, ferrierite, offretite, chabazite, gmelinite, erionite, ferrierite, mordenite and the ZSM family. When initially prepared, the cation in the crystalline molecular sieve is an alkali metal, typically sodium. This ion must be exchanged in sufficient proportion, usually 60% or greater, with an acidic ion such as a rare earth metal, e.g., lanthanum, praseodymium, hydrogen or some of the transition metals such as nickel, copper, chromium and the like. The substitution of various ions for the sodium ion alters the acidity of the crystalline molecular sieve, thus making it more reactive and catalytically effective for ring alkenylation of the aromatic amine.

The naturally occurring and synthetic zeolites used in the process normally have a silica to alumina molar ratio from about 2 to 25:1. However, if the silica to alumina ratio is low or acidity borders on the low side of that desired, the silica to alumina ratio and acidity of the zeolite may be altered by a technique called dealumination. In effect, the practice of dealumination decreases the alumina content in the zeolite thereby increasing the silica to alumina ratio. The removal of alumina from the internal structure affects acidity and may also enlarge the cage structure or pore size of zeolite to permit entry of an diffusion of larger molecules into its internal structure. Thus, one may be able to utilize a particular cation in a dealuminated zeolite, but not use the same cation in its non-dealuminated state. This is because the original cation may not have provided sufficient acidity for effecting ring alkenylation of the aromatic amine. Some of the techniques for dealumination include chelation, dehydration or acidification, the latter entailing the treatment of the zeolite with an inorganic acid. Techniques suited for dealumination of zeolites are well known.

Zeolites and crystalline molecular sieves are porous materials with the pores having generally uniform molecular dimensions. Cavities or cages are formed in this zeolite or molecular sieve and connected by channels of generally defined diameter. For the practice of this invention the pore diameter should be sufficiently large to permit the molecules to effectively enter the interior of the molecular sieve for reaction and to exit as final product. Typically, the pore size will range from about 6 to 15 Angstroms, but the size of a pore required for reaction can vary depending upon the product being produced. If conversion levels appear low for the particular catalyst, that level may be due to reactant diffusion resistance through the molecular sieve. If that is the case, a molecular sieve of slightly larger pore size should be tried.

Molecular sieves have been developed which have been defined as nonzeolites but have a cage structure that performs similarly to zeolites. In many cases, they contain alumina and silica in combination with other components, e.g., phosphorous, boron, germanium, titanium etc. In the alkenylation of aromatic amines as described here, they perform similarly to zeolites, and representative crystalline molecular sieves are described in U.S. Patent, 4,440,871; European Patent 124,119 and European Patent 121,232 and the subject matter of these patents incorporated by reference. Borosilicate and borogermanate zeolites, although not disclosed in these patents, possibly can also be used. For purposes of practicing this invention, i.e., in the production of alkenylated aromatic amines, molecular sieves are deemed equivalent to and included as catalyst material.

The preferred aromatic amines used in the alkenylation reaction are aniline and toluidine.

Diolefins useful in this invention are acyclic and cyclic conjugated dienes. Example of some dienes are 1,3-butadiene, isoprene, piperylene, 2,4-hexadiene, 2-phenyl-1,3-butadiene, cyclopentadiene, dicyclopentadiene, and methylcyclopentadiene.

Ring alkenylation of the aromatic amine is effected by reacting arylamine with the diolefin at temperatures ranging from about 100 to 250°C and preferably from about 140 to 220°C. The pressures will range from about 15 to 2000 psig and generally in the range of 100 to 1000 psig. It is common practice to alter the temperature and the pressure within the above ranges specified to optimize selectivity and conversion to the desired product. Mole ratios of olefins to aromatic amine used in the reaction will range from about 1:5 to 10:1 and the reaction time will generally be from about 2 to 48 hours when synthesized in an autoclave or within 0.05 to 6 hr -1, expressed as a liquid hourly space velocity (LHSV) for fixed bed continuous operation.

In the ring alkenylation of aromatic amines utilizing the solid acid catalyst systems, the diolefins, and particularly the olefins having conjugated unsaturation, tend to polymerize and generate substantial amount of by-product polymer. In many cases the combination of reactant and catalyst preclude the formation of alkenylated aromatic amines and substantially all of the olefin is converted to by-product polymer. Butadiene and cyclopentadiene are two offenders and both polymerize readily under the reaction conditions providing low yields of ring alkenylation. To avoid polymer production it is necessary to carry out the ring alkenylation of aromatic amine in the presence of a solvent which is inert to reaction with aromatic amine or the olefin and does not promote polymerization. Solvents which can be best utilized generally have a dielectric constant in the range of 1.5 to 3 and include paraffins such as pentane, hexane, octane, decane;

toluene and xylene, paraffinic naptha fractions, kerosene; and cycloparaffin hydrocarbons having from about 5 to 10 carbon atoms, e.g., cyclohexane and so forth.

In the alkenylation of an aromatic amine with conjugated diene, as for example butadiene or isoprene, the alkenyl substituent adds in such a way the olefinic bonds are $\beta$-$\gamma$ relative to the ring. The olefin is inert to common methods used for isomerization such as acid catalysis or catalysis by a Group VIII metal such as nickel or tungsten. The double bond of the substituent can be activated (isomerized) via isomerization placing it in conjugated relationship with the ring. Isomerization is accomplished by heating the alkenylation reaction product in the presence of base. Representative basic materials which can be used to effect isomerization are the alkali metal hedorxides, e.g., sodium or potassium hydroxide. The isomerization is facilitated through the use of solvents, e.g., a mixture of hydrocarbons and alcohols. Examples include a toluene/propanol mixture. Temperatures for isomerization range from 100 to 200°C and reaction times from 0.5 to 6.0 hrs.

An $\alpha$, $\beta$-unsaturated alkenyl arylamine is more reactive than the corresponding $\beta$,-$\gamma$-unsaturated alkenyl arylamine and, therefore, is useful as a synthon for the preparation of <u>bis</u>-dianiline monomers. For example, the preferred compositions may be used to make diamines (i.e., <u>bis</u>-dianilines) which are valuable monomers for preparing condensation polymers such as polyimides, polyamides, polyurethanes and epoxies. Representative formation of <u>bis</u>-dianiline monomers are shown below in the equations.

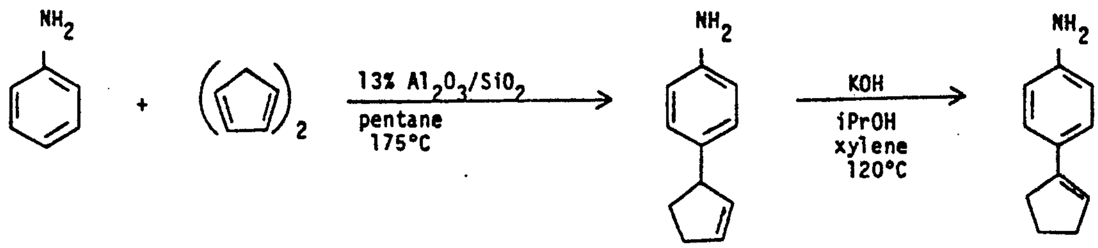


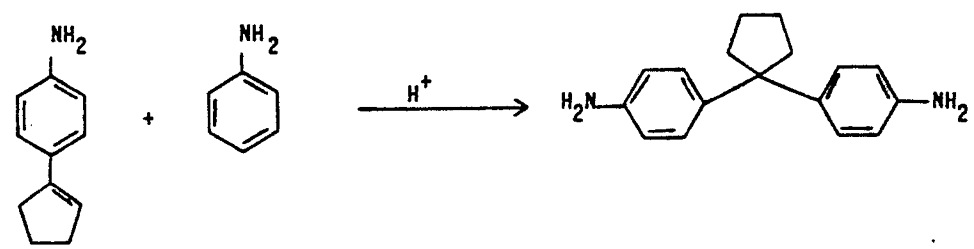


The compositions of this invention are also useful intermediated for capping low molecular weight condensation polymers so that they have chain ends terminated with olefinic unsaturation useful for post curing. The methods of curing such systems are well known and include ultraviolet light treatment and radical crosslinking initiated by peroxides or azo compounds. The text, <u>Polyurethanes Chemistry and Technology</u> by Saunders and Frisch, (1964) has several chapters relating to the utilization of polyurethanes having unsaturation therein; such chapters include applications for coatings, adhesives and fibers. The use of such end-capping will be useful for an array of condensation polymers inclusive of polyurethanes, polyimides, polyamides, polyesters and epoxies.

The styrenic double bond of the $\alpha$, $\beta$-unsaturated arylamine is substantially more reactive toward free radical addition reactions than the corresponding $\beta$, $\gamma$-unsaturated product. The $\beta$, $\gamma$-unsaturated arylamines contain allylic-benzylic hydrogen atoms which, upon hydrogen transfer, form a chain-terminating radical at the allylic-benzylic position.

In addition to formulating polyurethanes or polyurea elastomers, the pendant unsaturation in the alkenylated aromatic amines can be polymerized by conventional techniques with other polymerizable monomers, to enhance the physical properties of the elastomers systems. Typical monomers which may be polymerized with the pendant unsaturation include vinyl acetate, lower alkyl ($C_{1-6}$) esters of acrylic and methacrylic acid, vinyl chloride, vinylidine chloride, styrene, butadiene, isoprene and cyclopentadiene.

The following examples are provided to illustrate embodiments of the invention and are not intended to restrict the scope thereof. All parts are part by weight and all percentages are expressed as weight percent

unless otherwise specified.

Example 1

4-(But-2-enyl)aniline

A tubular reactor, consisting of a 0.5" I.D> 304 stainless steel tube and jacketed with a single element heater, was packed with 24cc (14.64g) of 12/18 mesh 13% $Al_2O_3$/87% $SiO_2$. The reactor pressure was brought up to 900 psi with accelerated aniline flow. The aniline flow was then reduced to 3.0cc/hr and the reactor temperature was increased to 175°C. Then pentane (19 cc/hr) and 1,3-butadiene (2.7cc/hr) were inctroduced downflow to the reactor. The relative molar ratio of feeds was 1 aniline; 1 butadiene; 5 n-pentane.

After an equilibration time of 16 hours, a sample was collected from the reactor and submitted to gas chromatographic analysis. The conversion of aniline was 34.4%. The product stream analyzed as follows:

| Product | % Mol. Selectivity |
|---|---|
| 2-(1-methylprop-2-enyl)aniline | 9.42 |
| 4-(but-2-enyl)aniline | 67.34 |
| 2-(1 methylprop-2-enyl)-4-(but-2-enyl)aniline | 16.16 |
| Unknowns | 7.08 |

Example 2

4-(But-1-enyl)aniline

To a 50 ml 2 neck round bottom flask, equipped with a sampling septum, magnetic stir bar, and short path distillation head, was added mixed xylenes (21.63g; 0.2041 mole), isopropanol (8.16g; 0.1360 mole), 85% potassium hydroxide (0.57g 0.0087 mole) and 4-(but-2-enyl)aniline (5.0g; 0.034 mole). The reaction mix was placed under a nitrogen atmosphere and stirred until the potassium hydroxide had dissolved. The reaction mixture was then heated to 114°C. Isopropanol was removed by distillation over a 25 minute period and then the oil bath temperature was raised to 170°C. The mixed xylene solvent was removed by distillation over a 45-minute time frame. The product, 3.03 g of 4-(but-1-enyl)aniline, was isolated by flash distillation and the structure verified by 'H NMR.

Example 3

4-(But-1-enyl)aniline via Mixture of Solid Acid and Solid Base Catalysts

A tubular reactor consisting of a 0.5" I.D., 304 stainless steel tube and jacketed with a single element heater was sequentially packed with 12 cc (7.78g) of CaO powder, a layer of vicor beads, and then 12cc (7.32g) of 12/18 mesh 13% $Al_2O_3$/87% $SiO_2$ catalyst. The catalyst bed was then activated under a nitrogen gas flow of 150cc/one-half hour increments. The bed was maintained at 200°C for one hour and then heated to 550°C in 100°c/one-half hour increments and then maintained at 550°C for two hours. Finally the temperature was adjusted to 175°C and kept under a flow of nitrogen overnight. The nitrogen flow was then discontinued and the reactor was brought up to 980 psi operating pressure with accelerated aniline flow. The aniline flow was then reduced to 3.0cc/hr and pentane (19.0cc/hr) and butadiene (2.7cc/hr) were introduced downflow to the reactor.

After 16 hours of operation, a sample was collected and submitted to gas chromtographic analysis. The overall conversion of aniline was less than 20%, but the selectivity of the product stream consisted of 30%

4-(but-1-enyl)aniline, 19% butylidene dianiline and 51% 4-(but-2-enyl)aniline.

The results of this experiment show that a mixture of acid/base catalysts can lead to the direct production of 4-(but-1-enyl)aniline from aniline and butadiene. The experiment also show that desirable butylidene dianilines may be formed via 4-(but-1-enyl)aniline.

Example 4

4-(Cyclopent-1-enyl)-aniline

A 50 ml 3 neck round bottom flask was equipped with a thermometer, sampling septum, and a short path distillation head. Isopropanol (4.00g; 0.0667 mole), mixed xylenes (11.00g; 0.1037 mole), 85% potassium hydroxide (0.25g; 0.0055 mole), and 4-(cyclopent-2-enyl)-aniline (2.77g; 0.0174 mole) were added to the 50 ml round bottom flask. The mixture was stirred via a magnetic stir bar under a nitrogen atmosphere. The solution was heated to 111°C over the course of one-half hour. The majority of the isopropanol was removed by distillation after 25 minutes and then the temperature of the reaction mix was raised to 140°C with the oil bath temperature at 160°C. After 70 minutes, 95% of the 4-(cyclopent-2-enyl)-aniline was converted to 4-(cyclopent-1-enyl)aniline. The product, 4-(cyclopent-1-enyl)aniline, was isolated by preparative gas chromatography and characterized by $^1$H NMR.

Example 5

Attempted Double Bond Isomerization Using Nickel Oxide/Tungsten Oxide Catalysts

4-(But-1-enyl)-aniline (3.0g) was heated with 1.0g nickel oxide/tungsten oxide to 200°C, 250°C and 280°C for eight hours at each temperature. The reaction was followed by gas chromatography. No double bond isomerization to the desired $\alpha$, $\beta$-unsaturated product was observed at any of the temperature. Some decomposition products were observed at 280°C.

Example 6

Attempted Double Bond Isomerization Using Strong Acid

To a 15 ml 2-neck r.b. flask containing 4.92g of 5-(cyclopent-2-enyl)-2,4-toluenediamine was added eight drops of trifluoromethanesulfonic acid. The flask was equipped with a condenser, placed under nitrogen atmosphere and heated at 190-200°C for three hours. Approximately 50% of the starting material dealkylated to form TDA while many other smaller amound of by-product were formed. The desired $\alpha$, $\beta$-unsaturated product was not produced.

**Claims**

1. An aromatic composition having at least one alkenyl substituent represented by the formula:

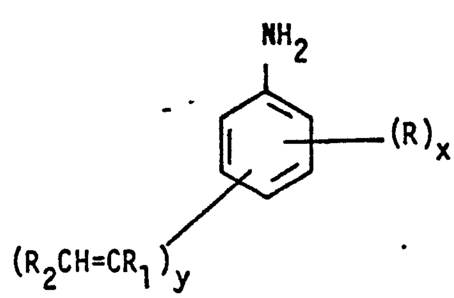

wherein R is H or $C_{1-5}$ alkyl; $R_1$ is H or $C_1$ methyl; $R_2$ is $C_{1-4}$alkyl; or $R_1$ or $R_2$ are combined as a 5 carbon member ring, x is 1 or 2, and y is 1 or 2 when R is H.

2. The composition of Claim 1 wherein y is 1 and R is H.

3. The composition of Claim 2 wherein $R_1$ is $C_2$ alkyl.

4. The composition of Claim 2 wherein $R_1$ is $C_1$ methyl.

5. The composition of Claim 2 wherein $R_2$ is $C_2$ and $R_1$ is $C_1$ methyl.

6. The composition of Claim 2 wherein $R_1$ and $R_5$ are combined into a 5 member ring.

7. The composition of Claim 1 wherein one R is $C_1$ alkyl, one R is H, and y is 1.

8. The composition of Claim 7 wherein $R_1$ is $C_1$ methyl and $C_2$ ethyl.

9. An aromatic amine composition having at least one akenyl substituent formed by first reacting an aromatic amine with a diene to form an alkenylated aromatic amine and then isomerizing the $\beta$-$\gamma$ double bond to an $\alpha$-$\beta$ double bond using a basic catalyst wherein said aromatic amine is aniline and said diene is cyclopentadiene, butadiene, or isoprene.

10. A process for making an alkenylated aniline composition having at least one alkenyl substituent and the double bonds are $\alpha$-$\beta$ which comprises:

(a) reacting aniline with a $C_4$-$C_8$ diene selected from the group consisting of isoprene, butadiene, cyclopentadiene, methyl cyclopentadiene, and methyl-1,3 pentadiene in the presence of an acidic solid-phase silica containing catalyst and an organic solvent having a dielectric constant in the range of 1.5-3 to form a $\beta$ -$\gamma$ unsaturated aniline derivative; and then,

(b) contacting the $\beta$ -$\gamma$ unsaturated aniline derivative with a base under conditions sufficient to isomerize the double bond to produce said aniline composition having an alkenyl substituent where the double bond is $\alpha$-$\beta$ to the ring.

11. The process of Claim 10 wherein said diene is butadiene.

12. The process of Claim 10 wherein said catalyst is silica-alumina and the base is an alkali metal hydroxide.

13. The process of Claim 10 wherein said diene is isoprene.

14. The process of Claim 10 wherein said diene is cyclopentadiene.